# EUROPEAN PATENT SPECIFICATION

(11) **EP 1 809 086 B1**
(45) Date of publication and mention of the grant of the patent: **02.12.2015**
(21) Application number: 05781591.2
(22) Date of filing: 05.09.2005
(51) Int. Cl.: H05K 9/00, G09F 9/00, A61B 1/00, A61B 1/04, A61B 5/06, G02F 1/1333

(54) **DISPLAY DEVICE**
ANZEIGEEINRICHTUNG
DISPOSITIF D'AFFICHAGE

(30) Priority: 08.09.2004 JP 2004261669
(43) Date of publication of application: 18.07.2007
(73) Proprietor: Olympus Corporation, Tokyo 151-0072 (JP)
(72) Inventor: KIMOTO, Seiichiro, Shibuya-ku, Tokyo 151-0072 (JP)
(74) Representative: von Hellfeld, Axel
(86) International application number: PCT/JP2005/016242
(87) International publication number: WO 2006/028047

(56) References cited:
- EP-A1- 1 196 018
- JP-A- 9 171 353
- JP-A- 10 322 625
- JP-A- 11 024 589
- JP-A- 11 038 893
- JP-A- 11 219 122
- JP-A- 2001 141 972
- JP-A- 2001 320 193
- JP-A- 2002 116 700
- JP-A- 2002 372 917
- JP-A- 2003 210 393
- JP-A- 2004 070 029
- JP-A- 2004 117 874
- JP-A- 2005 017 534

## Description

### TECHNICAL FIELD

The present invention relates to a display device having a display function capable of displaying input information on a screen, and more specifically, to a display device capable of preventing leakage of an electric wave from the outside into the device and leakage of an electromagnetic wave from the device to the outside.

### BACKGROUND ART

In recent years, in the field of an endoscope, a capsule endoscope as a swallowable endoscope having an imaging function and a radio communication function has been proposed, and a capsule endoscope system obtaining image data of the inside of the body of a subject with the capsule endoscope has been developed. The capsule endoscope is swallowed from the mouth of a subject for observation (examination) and travels in the subject body, for example, in organs such as the stomach and small intestine with the peristaltic movement of the organs while sequentially capturing images of the inside of the body of the subject in predetermined intervals such as 0.5-second intervals until the capsule endoscope is naturally discharged from the subject.

Intra-subject image data captured by the capsule endoscope for a period in which the capsule endoscope travels in the body of the subject is sequentially transmitted to an external receiving device by radio communication. The receiving device has a radio communication function and a memory function, sequentially receives intra-subject image data from the capsule endoscope via predetermined electric waves, and sequentially stores the received intra-subject image data into a memory. By carrying the receiving device, the subject can freely move also in the period since the subject swallows the capsule endoscope until the capsule endoscope is naturally discharged. After the capsule endoscope is naturally discharged, a doctor or a nurse obtains the intra-subject image data stored in the memory of the capsule endoscope by using a workstation or the like, and displays images of the inside of the subject, for example, images of organs based on the obtained intra-subject image data on the display of the workstation. By using the organ image and the like displayed on the display, the doctor or nurse can perform a diagnosis (see Patent Document 1, for example).

Generally, a receiving device used for such a capsule endoscope system has a radio communication unit for performing radio communication with a capsule endoscope and a storage unit for sequentially storing the obtained intra-subject image data. The receiving device also has a display unit such as a liquid crystal display for displaying input information, for example, information necessary for an examination on a subject, such as the patient ID and date and time of the examination. By using such a receiving device, for example, while the capsule endoscope travels in the subject, that is, the capsule endoscope captures images of the inside of the subject, a doctor, a nurse, or the subject can recognize information necessary for the examination on the subject, a reception state of the receiving device, and the like.
Patent Document 1: Japanese Patent Application Laid-Open No. 2003-19111

Document JP 9171353 A discloses an optical filter arrangement having an electrode structure to connect an optical filter to an external electrode. The optical filter is arranged in a display window of a casing. A conductive coating material is provided on an inner wall of the casing to block electromagnetic waves. The optical filter has a conductive mesh stuck to the rear surface of a transparent acrylic plate. The conductive mesh of the optical filter and the conductive coating material are electrically connected via an electrode.

Document JP 10322625 A discloses an optical filter mounted structure, wherein a conductive film is provided on an inner wall of a front frame which has a display window where an optical filter is arranged. Further, a transparent electrode is provided on the optical filter. The conductive film is electrically connected to the transparent electrode via a part of the conductive film on a surface of a blade spring formed by a cutout of a part of the inner side of the front frame.

Document JP 11219122 A discloses a plasma display panel unit having a shield case in a case which has a display window at a position where a plasma display panel is arranged. Further, a filter is arranged in the display window and a ground surface is provided inside the filter. The ground surface is electrically connected to the shield case.

Document JP 2002-116700 A discloses a method of forming an electrode part on the inner periphery of a translucent electromagnetic wave shielding plate. The plasma display panel unit comprises an electrode portion arranged on an inner wall of a casing which has a display window where a front plate is arranged. The front plate includes a conductive member arranged on an inner surface of a transparent film. The electrode portion of the casing is electrically connected to the conductive member via an electrode section.

### DISCLOSURE OF INVENTION

### PROBLEM TO BE SOLVED BY THE INVENTION

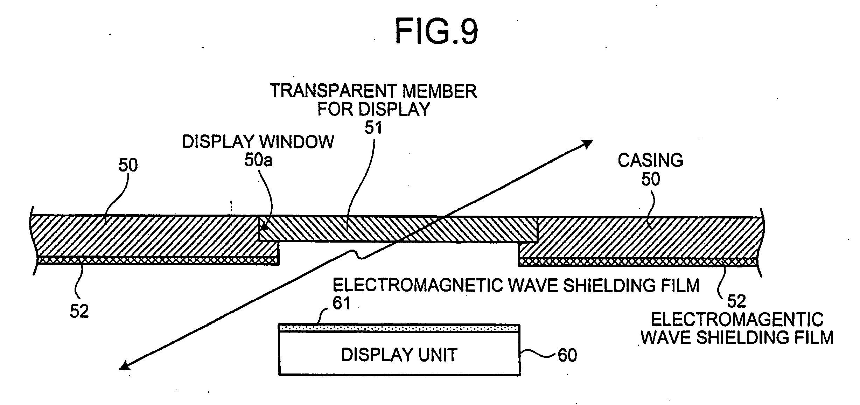

As shown in the above-described receiving device in the capsule endoscope system, a conventional display device having a display unit capable of displaying information obtained from the outside on a screen is provided with electromagnetic wave shielding unit on the inside of the casing in order to prevent electromagnetic interference. FIG. 9 is a partial schematic cross section of a main portion of a conventional display device. As shown in FIG. 9, in the conventional display device, a display window 50a is provided in a predetermined position in a casing 50, and a transparent member 51 for display is provided in a frame of the display window 50a. In a position corresponding to the transparent member 51 for display, a display unit 60 using a liquid crystal display (LCD) is disposed. The display unit 60 is fixed in the device by using a holder made of an insulating material or the like. On the inside of the casing 50, an electromagnetic wave shielding film 52 for blocking an electromagnetic wave entering from the outside or the inside of the device to the casing 50 is formed. On the surface of the display unit 60 facing the display window 50a, an electromagnetic wave shielding film 61 for blocking an electromagnetic wave entering the display 60 side via the transparent member 51 for display and an electromagnetic wave released from the display unit 60 is formed. In this case, an electromagnetic wave shielding film having optical transparency made of an indium-tin alloy oxide (ITO) or the like is formed as the electromagnetic wave shielding film 61.

In the conventional display device, however, as shown in FIG. 9, an air gap or an electric gap such as an insulating area (hereinbelow, written as a gap) exists between the electromagnetic wave shielding films 52 and 61 in many cases. It is difficult to form a conductive face covering all of gaps between the electromagnetic wave shielding films 52 and 61. Therefore, in some cases, the external electromagnetic wave or the electromagnetic wave generated in the device enters the device from the outside or leaks from the device to the outside via the gap between the electromagnetic wave shielding films 52 and 61, for example, as shown by the arrow in FIG. 9. Consequently, there is a problem that it is difficult to reliably prevent leakage of an electromagnetic wave between the outside and the inside of the device.

In particular, the receiving device having the display function used for the capsule endoscope system receives intra-subject image data from the capsule endoscope via weak electric waves, and there is the case such that the reception sensitivity deteriorates due to the electromagnetic wave leaked from the inside of the device. Therefore, it is demanded to reliably prevent leakage of an electromagnetic wave from the inside of the receiving device.

The present invention has been achieved in consideration of the above circumstances, and an object of the invention is to provide a display device capable of reliably preventing leakage of an external electromagnetic wave to the inside of the device and leakage of an electromagnetic wave generated in the device to the outside.

### MEANS FOR SOLVING PROBLEM

The present invention provides a display device according to claim 1.

A display device according to one aspect of the present invention includes a shielding film provided on an inner wall of a casing having a display window, the shielding film blocking an electromagnetic wave via the casing; and an optically transparent shielding film provided on an inner surface of a transparent film for display provided for the display window, the optically transparent shielding film having an optical transparency, blocking an electromagnetic wave via the transparent film for display, and being electrically connected to the shielding film.

The display device according the present invention may further include a conductive member that covers a gap between the shielding film and the optically transparent shielding film and electrically connects the shielding film and the optically transparent shielding film.

In the display device according to the present invention, the conductive member may be a fixing member for fixing the transparent film for display to the display window.

In the display device according to the present invention, the transparent film for display may include a first transparent film which is fit in a frame of the display window; and a second transparent film which is fixed to the display window from an inside of the first transparent film and covers the display window, and the optically transparent shielding film may be provided on a back side of a side on which the second transparent film is fixed to the display window.

In the display device according to the present invention, the optically transparent shielding film may be provided on the inner wall of the transparent film for display.

In the display device according to present invention, the optically transparent shielding film may be an indium-tin alloy oxide film.

In the display device according to the present invention, the optically transparent shielding film may be a metal mesh.

In the display device according to present invention may include a receiving unit that receives image data of an inside of a subject from a capsule endoscope in the subject via a predetermined electric wave.

### EFFECTS OF THE INVENTION

The present invention produces an effect such that a display device can be realized in which a series of conductive surfaces covering the inside of a casing can be formed, and leakage of an external electromagnetic wave to the inside of the casing and leakage of an electromagnetic wave generated on the inside of the casing to the outside can be prevented with reliability.

### BRIEF DESCRIPTION OF DRAWINGS

FIG. 1 is a schematic diagram showing an example of the configuration of a capsule endoscope system using a receiving device having a display function as an embodiment of the present invention;
FIG. 2 is a block diagram showing an example of the configuration of the receiving device;
FIG. 3 is a schematic side view showing a side-sectional structure of the receiving device;
FIG. 4 is a schematic view showing the appearance of a portion around a display window in the receiving device;
FIG. 5 is a schematic cross section taken along line A-A of the receiving device shown in FIG. 4;
FIG. 6 is a schematic side view showing a side-sectional structure of a portion around a display window in a receiving device as a modification of the embodiment of the invention;
FIG. 7 is a schematic cross section of a main portion illustrating a configuration in which an electromagnetic wave shielding film as a single film having optical transparency and an electromagnetic wave shielding film on the inner wall of a casing are electrically connected to each other;
FIG. 8 is a schematic cross section of a main portion illustrating a configuration in which a single body of an electromagnetic wave shielding film having optical transparency and an electromagnetic wave shielding film on the inner wall of a casing are electrically connected to each other via a conductive adhesive; and
FIG. 9 is a partial schematic cross section of a main portion of a conventional display device.

### EXPLANATIONS OF LETTERS OR NUMERALS

- 1, 21: RECEIVING DEVICE
- 2a to 2h: RECEIVING ANTENNA
- 3: INPUT UNIT
- 4, 60: DISPLAY UNIT
- 5: ANTENNA SELECTOR
- 6: RECEIVING CIRCUIT
- 7: SIGNAL PROCESSOR
- 8: A/D CONVERTER
- 9: CONTROL UNIT
- 10: STORAGE UNIT
- 11: POWER SUPPLY UNIT
- 12, 50: CASING
- 12a: DISPLAY WINDOW
- 13: CIRCUIT BOARD
- 14, 22, 51: TRANSPARENT MEMBER FOR DISPLAY
- 15, 16a, 23, 31, 41, 52, 61: ELECTROMAGNETIC WAVE SHIELDING FILM
- 16: ELECTROMAGNETIC SHIELD
- 17, 24: GASKET
- 32: ADHESIVE
- 42: CONDUCTIVE ADHESIVE
- 100: SUBJECT
- 101: CAPSULE ENDOSCOPE
- 102: WORKSTATION
- 103: PORTABLE RECORDING MEDIUM

### BEST MODE FOR CARRYING OUT THE INVENTION

Preferred embodiments of a display device according to the present invention will be described in detail hereinbelow with reference to the drawings. In the following, as an example of the display device according to the invention, a receiving device of a capsule endoscope system having a radio communication function and a display function is illustrated.

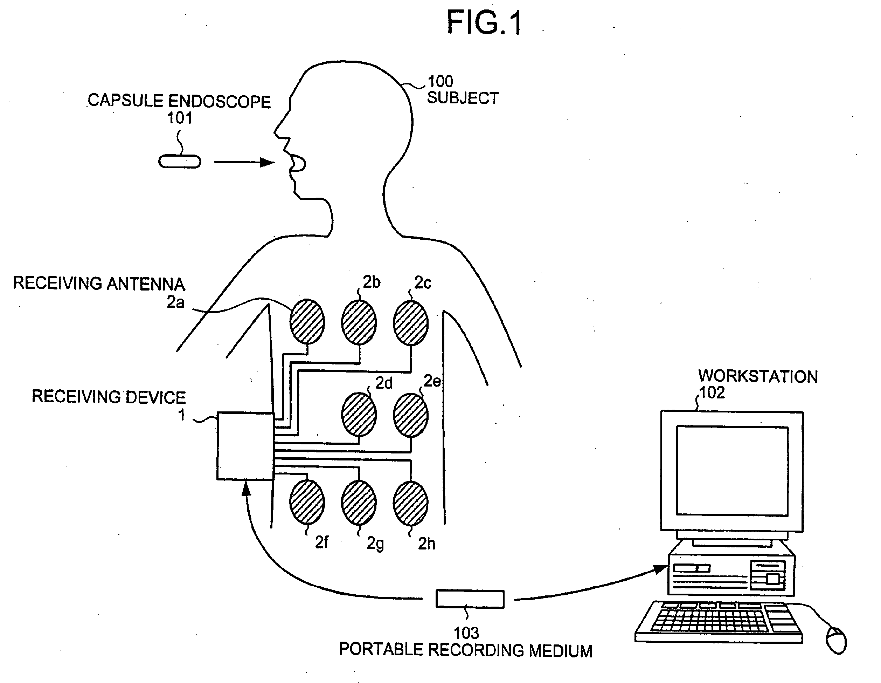

FIG. 1 is a schematic diagram illustrating an example of the configuration of a capsule endoscope system using the receiving device according to the embodiment of the invention. As shown in FIG. 1, the capsule endoscope system has: a capsule endoscope 101 which travels along a path in a subject 100 and captures images of the inside of the subject 100; a receiving device 1 for receiving image data transmitted from the capsule endoscope 101; a workstation 102 for displaying an image of the inside of the subject 100 captured by the capsule endoscope 101; and a portable recording medium 103 for delivering information between the receiving device 1 and the workstation 102.

The capsule endoscope 101 has an imaging function capable of capturing images of the inside of a subject and a radio communication function of transmitting image data obtained by capturing images of the inside of the subject to the outside. The capsule endoscope 101 is swallowed by the mouth of the subject 100, travels in the stomach in the subject 100, and travels in the body cavity with the peristaltic movement of the digestive organs. Simultaneously, the capsule endoscope 101 sequentially captures images in the body cavity of the subject 100, and the image data of the inside of the subject 100 is sequentially received by the receiving device 1.

The workstation 102 is provided to display the images of the inside of the subject captured by the capsule endoscope 101, and displays the images of the organs and the like in the subject 100 on the basis of the data obtained by the portable recording medium 103. The workstation 102 also has a process function for allowing a doctor or a nurse to perform a diagnosis on the basis of the images of the organs and the like in the subject by the capsule endoscope 101.

The portable recording medium 5 has a structure which is removably inserted to the receiving device 1 and the workstation 102 and can output or record information when inserted in the receiving device 1 and the workstation 102. Specifically, while the capsule endoscope 101 travels in the body cavity of the subject 100, the portable recording medium 103 is inserted in the receiving device 1 and the receiving device 1 sequentially stores the image data of the inside of the subject 100 received from the capsule endoscope 101. After the capsule endoscope 101 is discharged from the subject 100, the portable recording medium 103 is taken out from the receiving device 1 and inserted in the workstation 102. In this case, the workstation 102 can read data recorded by the receiving device 1 such as image data of the inside of the subject 100 from the inserted portable recording medium 103.

As the portable recording medium 103, various recording media such as CompactFlash®, SmartMedia, MemoryStick, MultimediaCard, and SD Memory Card are used. By delivering data between the receiving device 1 and the workstation 102 via the portable recording medium 103, the subject 100 can move freely in a state where the subject 100 carries the receiving device 1 even when the capsule endoscope 101 is traveling in the subject 100, different from the case where the receiving device 1 and the workstation 102 are connected to each other via a communication cable or the like.

The receiving antennas 2a to 2h are realized by, for example, loop antennas and receive radio signals transmitted from the capsule endoscope 101. The receiving antennas 2a to 2h are disposed in predetermined positions on the body surface of the subject 100, for example, positions corresponding to the path of the capsule endoscope 101. The receiving antennas 2a to 2h are electrically connected to the receiving device 1 and transmit a radio signal received from the capsule endoscope 101 to the receiving device 1. The receiving device 1 sequentially receives image data of the inside of the subject 100 from the capsule endoscope 101 via predetermined electric waves transmitted/received between the capsule endoscope 101 and the receiving antennas 2a to 2h.

The receiving antennas 2a to 2h may be disposed in predetermined positions in a jacket to be put on the subject 100. In this case, the receiving antennas 2a to 2h are disposed in predetermined positions of the subject 100 when the subject 100 wears the jacket. It is sufficient to dispose a plurality of receiving antennas on the subject 100 and the number of the receiving antennas is not limited to eight. By disposing the plurality of receiving antennas on the subject 100, the receiving device 1 can receive image data from the capsule endoscope 101 via the antenna in a position adapted to receive a radio signal in accordance with the position of the capsule endoscope 101 in the subject 100.

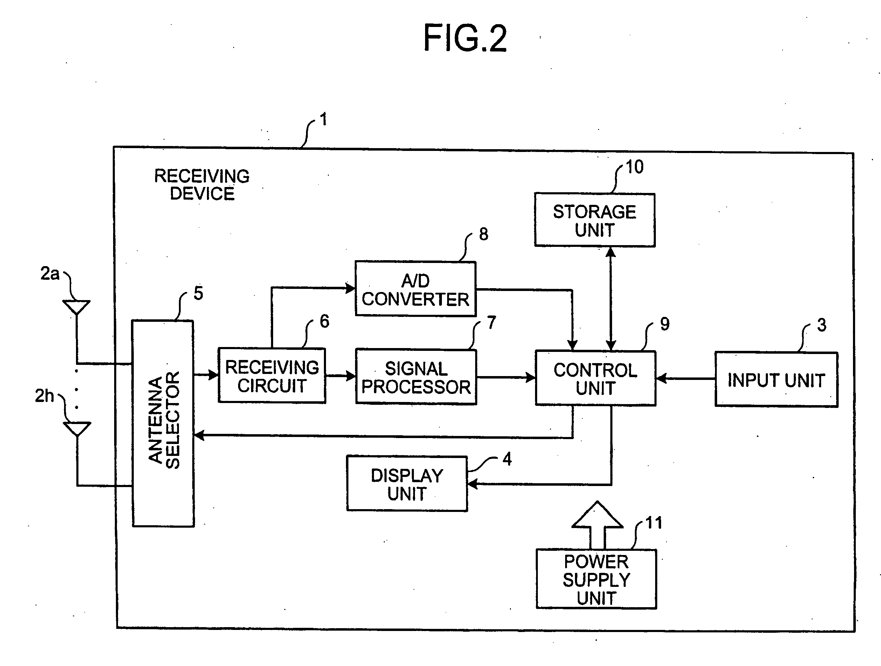

The receiving device 1 has the display function of displaying input information on the screen and the radio communication function of receiving a radio signal from the capsule endoscope 101, and performs a process of receiving a radio signal from the capsule endoscope 101 via the receiving antennas 2a to 2h. FIG. 2 is a block diagram showing an example of the configuration of the receiving device 1. As shown in FIG. 2, the receiving device 1 has: an input unit 3 for entering information necessary for observation (examination) of the inside of a subject, and the like; a display unit 4 for displaying the information supplied by the input unit 3 and the like on a screen; an antenna selector 5 for selecting a receiving antenna suitable for receiving a radio signal from the receiving antennas 2a to 2h; a receiving circuit 6 for performing a process such as demodulation or the like on the radio signal received from the capsule endoscope 101 via any one of the receiving antennas 2a to 2h selected by the antenna selector 5; and a signal processor 7 for extracting image data of the inside of the subject 100 or the like from the radio signal subjected to the process in the receiving circuit 6. The receiving device 1 has a control unit 9 for performing a predetermined control on an output of the information extracted by the signal processor 7, and a storage unit 10 for storing the extracted information such as image data. The receiving device 1 also has the A/D converter 8 for converting an analog signal supplied from the receiving circuit 6 to a digital signal, and a power supply unit 11 for supplying a drive power of each of the components of the receiving device 1.

The input unit 3 is realized by using a plurality of input keys, a touch panel, or the like, and enters information according to an input operation of an operator such as a patient as the subject, a doctor, a nurse, or the like to the control unit 9. For example, the input unit 3 enters information necessary for an examination on the subject such as the patient ID specifying a patient to be examined or an examination ID specifying an examination to be executed to the control unit 9 in accordance with an input operation of the operator. The input unit 3 enters instruction information on various operations of the receiving device 1, for example, instruction information of starting or finishing reception of a radio signal from the capsule endoscope 101 to the control unit 9 in accordance with an input operation of the operator.

The display unit 4 is realized by using a thin display such as a liquid crystal display (LCD), an organic EL panel, or a plasma display panel, and displays the information supplied from the control unit 9 under control of the control unit 9. For example, the display unit 4 displays information necessary for an examination on the subject, supplied from the input unit 3 such as the patient ID specifying a patient to be examined or examination ID specifying an examination to be conducted under control of the control unit 9. The display unit 4 also displays information indicative of a reception state of the receiving device 1, for example, information indicating whether or not a radio signal could have been received from the capsule endoscope 101 without any problem under control of the control unit 9. The display unit 4 may sequentially display images of the inside of the subject 100 on the basis of the image data received from the capsule endoscope 101 under control of the control unit 9.

The antenna selector 5 functions to select a receiving antenna suitable for receiving a radio signal from the capsule endoscope 101 from the plurality of receiving antennas 2a to 2h disposed on the subject 100. Specifically, the antenna selector 5 selects a predetermined receiving antenna from the receiving antennas 2a to 2h under control of the control unit 9, and receives a radio signal from the capsule endoscope 101 via the selected receiving antenna. In this case, the antenna selector 5 transmits the received radio signal to the receiving circuit 6.

The receiving circuit 6 functions to perform a predetermined process such as demodulation on a radio signal received from the capsule endoscope 101 via the receiving antenna selected by the antenna selector 5. In this case, the receiving circuit 6 performs a predetermined process such as demodulation on the radio signal supplied from the antenna selector 5, and transmits the radio signal subjected to the predetermined process to the signal processor 7. The receiving circuit 6 transmits to the A/D converter 8 an analog signal corresponding to intensity of the radio signal supplied from the antenna selector 5.

The signal processor 7 extracts predetermined information from the radio signal subjected to the predetermined process in the receiving circuit 6. For example, in the case where a radio signal received by the receiving device 1 is transmitted from an electronic device having the imaging function, the signal processor 7 extracts image data from the radio signal supplied from the receiving circuit 6. In other words, when the receiving device 1 receives a radio signal from the capsule endoscope 101, the signal processor 7 extracts image data of the inside of the subject 100 by the capsule endoscope 101 from the radio signals supplied from the receiving circuit 6.

The control unit 9 is realized by using a central processing unit (CPU) for executing various processing programs, a ROM in which the various processing programs and the like are stored in advance, and a RAM for storing computation parameters of processes, information supplied from the input unit 3, image data supplied from the signal processor 7, and the like. The control unit 9 controls the operations of the components of the receiving device 1 including the antenna selecting operation of the antenna selector 5. Specifically, the control unit 9 transfers and stores information supplied from the input unit 3 or the signal processor 7 to the storage unit 10. The control unit 9 also determines a receiving antenna to be used on the basis of a digital signal supplied from the A/D converter 8, that is, a digital signal obtained by A/D converting an analog signal corresponding to the reception intensity of the radio signal, for example, a received signal strength indicator (RSSI), and controls the antenna selector 5 to select the determined receiving antenna.

The control unit 9 determines the reception state of the receiving device 1 on the basis of a signal supplied from the signal processor 7 or the A/D converter 8, for example, a digital signal corresponding to reception intensity, and allows information indicative of the determined reception state to be displayed on the display unit 4. Further, the control unit 9 controls the operation of the components on the basis of the instruction information supplied from the input unit 3. For example, when instruction information for starting reception of a radio signal from the capsule endoscope 101 is received from the input unit 3, the control unit 9 performs a control of starting the operation of receiving the radio signal on the basis of the instruction information. In the case where instruction information of finishing reception of a radio signal from the capsule endoscope 101 is supplied from the input unit 3, the control unit 9 performs a control of finishing the operation of receiving the radio signal on the basis of the instruction information.

The storage unit 10 stores information supplied from the control unit 9, for example, image data extracted by the signal processor 7 or information supplied from the input unit 3 under control of the control unit 9. As a specific configuration of the storage unit 10, the storage unit 10 itself may store information by providing a RAM, a flash memory, or the like. In the embodiment, the portable recording medium 103 can be removably inserted in the storage unit 10. Under control of the control unit 9, the storage unit 10 functions to write information supplied from the control unit 9 to the portable recording medium 103. In this case, the storage unit 10 may read information from the inserted portable recording medium 103 and transmit the read information to the control unit 9 under control of the control unit 9.

The power supply unit 11 supplies drive power to each of the components of the receiving device 1 also in the case where the receiving device 1 is carried by the subject 100. Examples of the power supplies 11 are a dry cell, a lithium ion secondary battery, and a nickel hydrogen battery. The power supply unit 11 may be of a rechargeable type.

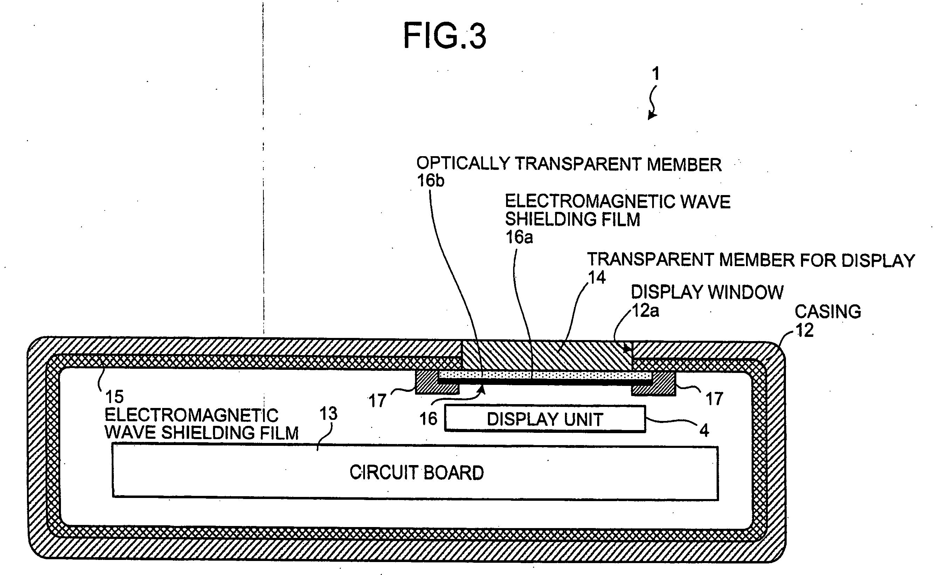

Next, the structure of the receiving device 1 as an embodiment of the invention will be described in detail. FIG. 3 is a schematic side section illustrating a side-sectional structure of the receiving device 1. As shown in FIG. 3, in the receiving device 1, the display unit 4 and a circuit board 13 are assembled in a casing 12. The circuit board 13 is provided with the antenna selector 5, receiving circuit 6, signal processor 7, A/D converter 8, control unit 9, and storage unit 10. Although not shown in FIG. 3, a plurality of input keys constructing the input unit 3 are provided in predetermined positions in the casing 12 and are electrically connected to the control unit 9 via the circuit board 13. The power supply unit 11 is provided in a predetermined position in the casing 12 and electrically connected to the circuit board 13.

The casing 12 is made of an insulating material such as polycarbonate resin or ABS resin and forms a package of the receiving device 1. In the casing 12, as shown in FIG. 3, a display window 12a is formed in a predetermined position, and a transparent member 14 for display is provided in the frame of the display window 12a. As shown in FIG. 3, the display unit 4 is disposed in the position corresponding to the display window 12a and electrically connected to the control unit 9 via the circuit board 13. In this case, the display unit 4 may be fixed to the circuit board 13 or fixed to the casing 12 by using a supporting member such as a holder. The transparent member 14 for display is realized by using a resin or glass having optical transparency, allows the screen display of the display unit 4 to be visually recognized from the outside, and protects the display unit 4 from the outside.

On the inner wall of the casing 12, an electromagnetic wave shielding film 15 is formed which blocks external electromagnetic waves released from the outside to the casing 12 side or electromagnetic waves released on the inside of the device, for example, the display unit 4 or the circuit board 13 to the casing 12 side. The electromagnetic wave shielding film 15 is realized by forming a thin film of a metal such as copper, nickel, aluminum, or chromium on the almost entire surface of the inner wall of the casing 12, and is electrically connected to the ground of the circuit board 13.

On the other side, an electromagnetic shield 16 having a plate shape or a film state is attached via the electromagnetic wave shielding film 15 to the inner wall periphery around the display window 12a in the casing 12. The electromagnetic shield 16 is realized by forming an electromagnetic wave shielding film 16a having optical transparency such as an indium tin alloy oxide film (ITO film) on one of faces of an optically transparent member 16b such as a resin plate or a resin film having optical transparency. The optically transparent member 16b protects the electromagnetic wave shielding film 16a formed on its surface. Consequently, a scratch is suppressed, and further, damage or breakage in the electromagnetic wave shielding film 16a is also suppressed.

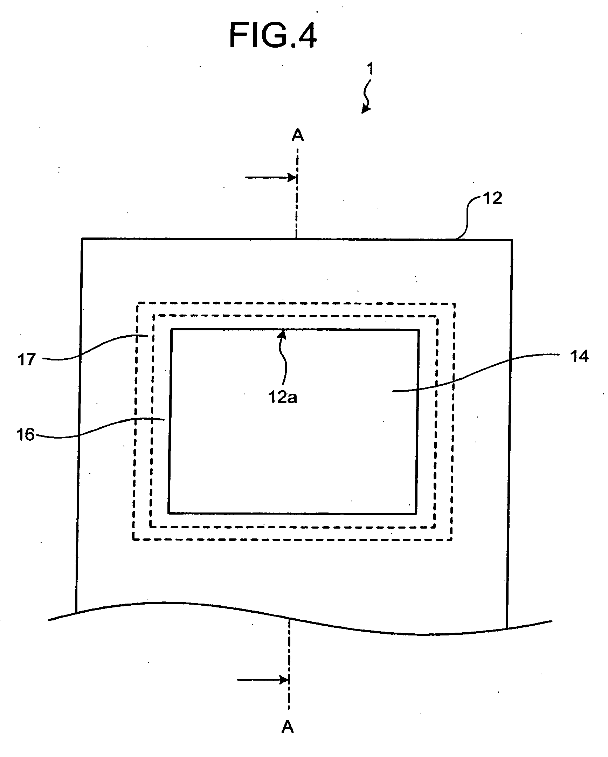

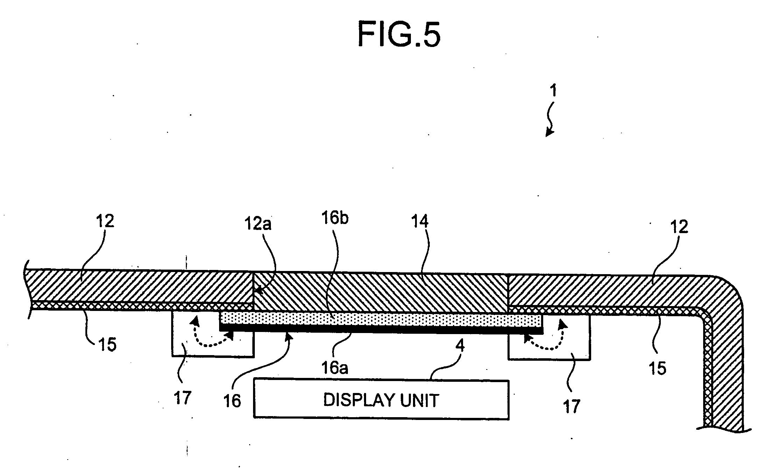

A gasket 17 is provided near the periphery of the electromagnetic shield 16 attached. FIG. 4 is a schematic view illustrating the appearance of the device around the display window 12a in the receiving device 1. FIG. 5 is a schematic cross section taken along line A-A of the receiving device 1 of FIG. 4 and schematically illustrating a side-sectional structure of the portion around the display window 12a in the receiving device 1.

As described above, the electromagnetic shield 16 has the electromagnetic wave shielding film 16a and the optically transparent member 16b. As shown in FIG. 4, the electromagnetic shield 16 is attached to the inner wall of the casing 12 so as to cover the display window 12a. Specifically, as shown in FIG. 5, the electromagnetic shield 16 is attached to the inner wall of the casing 12 by surface contact between the periphery of the optically transparent member 16b and the inner wall periphery around the display window 12a of the casing 12 via an adhesive or sticker in a state where the electromagnetic wave shielding film 16a faces the display unit 4 side. In this case, each of the transparent member 14 for display and the optically transparent member 16b is constructed by a transparent film for display through which the screen display of the display unit 4 can be visually recognized from the outside. The electromagnetic shield 16 for display may be fixed to the transparent member 14 for display by using a known transparent adhesive sheet or transparent adhesive. Preferably, the electromagnetic shield 16 is attached to the inner wall of the casing 12 in a state where a gap is formed between itself and the transparent member 14 for display. It can prevent the more effect due to the contact between the transparent member 14 for display and the electromagnetic shield 16. In the case of making the electromagnetic shield 16 fixed or in contact with the transparent member 14 for display, it is desirable to take a countermeasure of, for example, making the contact surface of the transparent member 14 for display or the electromagnetic shield 16 rough.

As the resin of the optically transparent member 16b, polymethylpentene (PMP), polystyrene (PS), polycarbonate (PC), or polyethylene terephthalate (PET) may be used. The optically transparent member 16b may be made of glass having optical transparency.

The gasket 17 is composed of a conductive member, and as shown in FIG. 4, is provided in a frame shape along the periphery of the electromagnetic shield 16 attached to the inner wall periphery around the display window 12a in the casing 12. In this case, as shown in FIG. 5, the gasket 17 is provided so as to bury an attachment portion between the inner wall of the casing 12 and the electromagnetic shield 16, that is, the inner wall periphery around the display window 12a in the casing 12 and the periphery of the electromagnetic shield 16. By the gasket 17 provided in such a manner, the periphery of the electromagnetic shield 16 can be reliably fixed to the inner wall periphery around the display window 12a in the casing 12. At the same time, the gasket 17 can realize moisture barrier property of the attachment portion for the electromagnetic shield 16.

The electromagnetic shield 16 is attached to the inner wall of the casing 12 in a state where the electromagnetic wave shielding film 16a faces the display unit 4 side as described above. The gasket 17 buries the inner wall periphery around the display window 12a in the casing 12 and the periphery of the electromagnetic shield 16, thereby enabling the gap between the electromagnetic wave shielding films 15 and 16a to be covered with the periphery of the electromagnetic shield 16 along the periphery of the electromagnetic shield 16. In this case, the gasket 17 forms a conductive surface covering the gap between the electromagnetic films 15 and 16a along the periphery of the electromagnetic shield 16. The conductive surface formed by the gasket 17 is a border surface that connects the conductive surface of the electromagnetic wave shielding film 15 and the conductive surface of the electromagnetic wave shielding film 16a. Therefore, the electromagnetic wave shielding films 15 and 16a and the gasket 17 can form a series of conductive surfaces in which the conductive surface formed by the electromagnetic wave shielding film 15 and the conductive surface formed by the electromagnetic wave shielding film 16a are continuous via the conductive surface formed by the gasket 17 so as to cover the entire inside of the casing 12.

The electromagnetic wave shielding film 16a forming part of the series of conductive surfaces is electrically connected to the ground of the circuit board 13 via the gasket 17 and the electromagnetic wave shielding film 15. The electromagnetic wave shielding film 16a electrically connected to the ground can block external electromagnetic waves released from the outside to the transparent member 14 for display and electromagnetic waves released on the inside of the device, that is, electromagnetic waves released from the display unit 4 or the circuit board 13 to the transparent member 14 side for display. Since the gasket 17 forming part of the series of conductive surfaces is connected to the ground of the circuit board 13 via the electromagnetic wave shielding film 15, in a manner similar to the case of the electromagnetic wave shielding film 16a, the electromagnetic waves can be blocked. Further, the electromagnetic wave shielding film 15 forming part of the series of conductive surfaces can block external electromagnetic waves released from the outside to the casing 12 side and electromagnetic waves released from the inside of the device to the casing 12 side. Therefore, by forming the series of conductive surfaces by the electromagnetic wave shielding films 15 and 16a and the gasket 17, leakage of the external electromagnetic waves to the inside of the receiving device 1 and leakage of the electromagnetic waves generated in the receiving device 1 to the outside can be prevented with reliability. In this case, the receiving device 1 can receive a radio signal from the capsule endoscope 101 without being hindered by the electromagnetic waves generated in the receiving device 1, and accordingly, reception sensitivity of the receiving device 1 can be improved. Further, the receiving device 1 can prevent an adverse influence such as erroneous operation caused by the external electromagnetic waves.

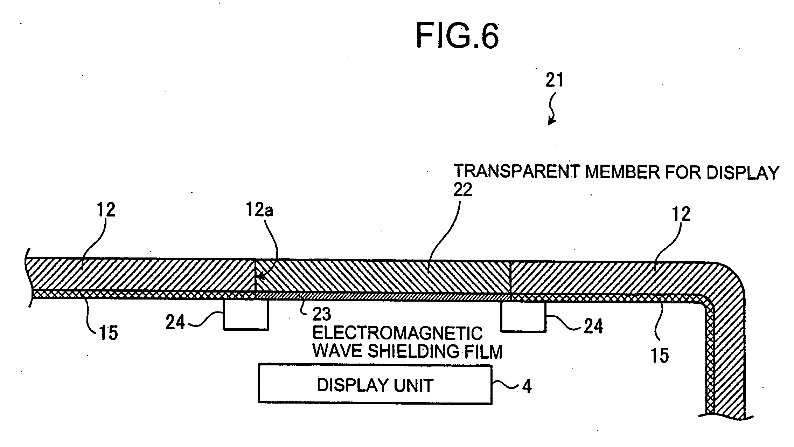

Although the electromagnetic shield 16 having a plate or film shape is attached to the inner wall of the casing 12 in the embodiment, the invention is not limited to the configuration. A transparent member for display having a surface on which an electromagnetic wave shielding film having optical transparency is formed may be provided for the display window 12a in the casing 12. FIG. 6 is a schematic side view showing a side-sectional structure of a portion around a display window in a receiving device as a modification of the embodiment of the invention. A receiving device 21 has a transparent member 22 for display in place of the transparent member 14 for display in the receiving device 1, and a gasket 24 in place of the gasket 17. In the receiving device 21, the electromagnetic shield 16 is not provided but an electromagnetic wave shielding film 23 having optical transparency is formed on one surface of the transparent member 22 for display. The other configurations are similar to those of the foregoing embodiment, and the same reference numeral is given to the same component.

The transparent member 22 for display is realized by using the resin or glass having optical transparency, enables the screen display of the display unit 4 to be visibly recognized from the outside, and functions as a transparent film for display that protects the display unit 4 from the external force. An electromagnetic wave shielding film 23 having optical transparency is formed on a predetermined surface of the transparent member 22 for display. The electromagnetic wave shielding film 23 is formed by depositing an ITO film or applying an ITO film on which an ITO layer is formed to the surface of the transparent member 22 for display. The transparent member 22 for display is attached to the display window 12a so that the electromagnetic wave shielding film 23 faces the display unit 4 side as shown in FIG. 6. As the resin of the optically transparent member 22 for display, polymethylpentene (PMP), polystyrene (PS), polycarbonate (PC), or polyethylene terephthalate (PET) may be used.

The gasket 24 is a conductive member and provided in a frame shape along the contact interface between the frame of the display window 12a and the transparent member 22 for display. In this case, as shown in FIG. 6, the gasket 24 is provided so as to bury the inner wall periphery around the display window 12a in the casing 12 and the periphery of the electromagnetic wave shielding film 23 in the transparent member 22 for display. The gasket 24 provided in such a manner can realize moisture barrier property of a contact interface between the frame of the display window 12a and the transparent member 22 for display and can close the gap between the electromagnetic wave shielding film 15 and the electromagnetic wave shielding film 23 along the contact interface. In this case, the gasket 24 forms a conductive surface covering the gap between the electromagnetic films 15 and 23 along the contact interface. Therefore, in a manner similar to the case of the foregoing embodiment, the electromagnetic wave shielding films 15 and 23 and the gasket 17 can form a series of conductive surfaces electrically connected to the ground of the circuit board 13 and entirely covering the inside of the casing 12. By forming the series of conductive surfaces, in a manner similar to the case of the foregoing embodiment, leakage of the external electromagnetic waves to the inside of the receiving device 21 and leakage of the electromagnetic waves generated in the receiving device 21 to the outside can be prevented with reliability. In this case, the receiving device 21 can receive a radio signal from the capsule endoscope 101 without being hindered by the electromagnetic waves generated in the receiving device 21, and accordingly, reception sensitivity of the receiving device 21 can be improved. Further, the receiving device 21 can prevent an adverse influence such as erroneous operation caused by the external electromagnetic waves.

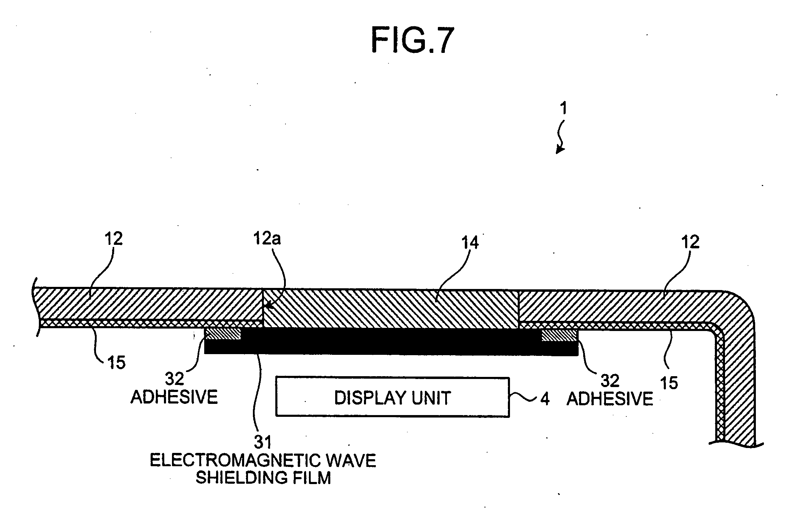

In the embodiment and its modification of the invention, the electromagnetic wave shielding film having optical transparency provided on the inner wall of the optically transparent member such as a transparent member for display and the electromagnetic wave shielding film provided on the inner wall of the casing are electrically connected to each other via a conductive member such as a gasket. However, the invention is not limited to the configuration. Alternatively, an electromagnetic wave shielding film having optical transparency which is not formed on the inner wall of the optically transparent member but is formed as a single film and an electromagnetic wave shielding film provided on the inner wall of the casing may be electrically connected to each other. FIG. 7 is a schematic cross section of a main portion of the receiving device 1 having a configuration in which an electromagnetic wave shielding film as a single film having optical transparency and an electromagnetic wave shielding film on the inner wall of a casing are electrically connected to each other. In the receiving device 1, an electromagnetic wave shielding film 31 is provided in place of the electromagnetic shield 16, and the electromagnetic wave shielding film 31 and the electromagnetic wave shielding film 15 are electrically connected to each other without using the gasket 17.

As shown in FIG. 7, the electromagnetic wave shielding film 31 having optical transparency formed as a single film is realized by a conductive film having optical transparency such as an ITO film, and its periphery and the electromagnetic wave shielding film 15 are in surface-contact with each other along the frame of the display frame 12a. In such a manner, the electromagnetic wave shielding films 31 and 15 are electrically connected to each other so as to form a series of conductive surfaces entirely covering the inside of the casing 12. In this case, as shown in FIG. 7, the electromagnetic wave shielding film 31 is attached to the inner wall of the casing 12 by, for example, an insulating adhesive 32 without using the gasket 17. The adhesive 32 does not hinder surface contact accompanying electric connection between the electromagnetic wave shielding films 15 and 31.

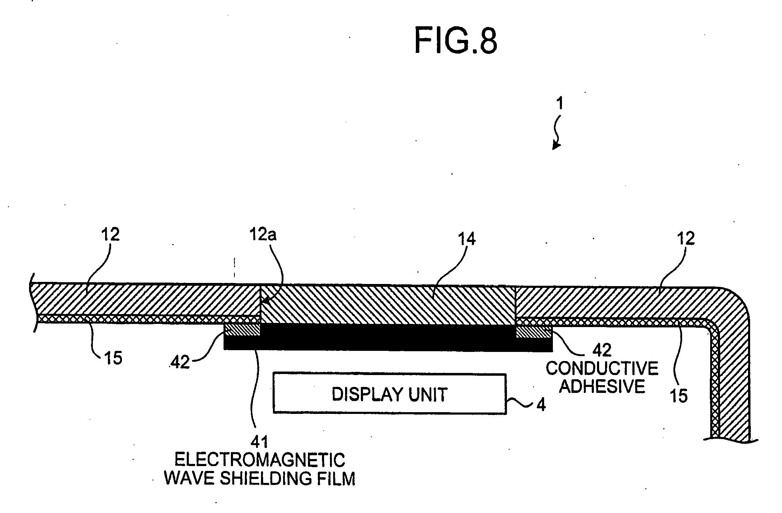

Alternatively, the electromagnetic wave shielding film having optical transparency formed as a single film may be attached to the casing via a conductive adhesive and electrically connected to the electromagnetic wave shielding film on the inner wall surface of the casing. FIG. 8 is a schematic cross section of a main portion of the receiving device 1 having a configuration in which the electromagnetic wave shielding film having optical transparency formed as a single film and the electromagnetic wave shielding film on the inner wall of the casing are electrically connected to each other via a conductive adhesive. In the receiving device 1, an electromagnetic wave shielding film 41 is provided in place of the electromagnetic shield 16, and the electromagnetic wave shielding film 41 and the electromagnetic wave shielding film 15 are electrically connected to each other via a conductive adhesive 42 in place of the gasket 17.

As shown in FIG. 8, the electromagnetic wave shielding film 41 having optical transparency formed as a single film is realized by a conductive film having optical transparency such as an ITO film, and its periphery and the electromagnetic wave shielding film 15 are in surface-contact with each other along the frame of the display frame 12a. In such a manner, the electromagnetic wave shielding film 41 is attached to the inner wall face of the casing 12 and electrically connected to the electromagnetic wave shielding film 15 so as to form a series of conductive surfaces entirely covering the inside of the casing 12.

In the embodiment and its modification of the invention, the ITO film is used as the electromagnetic wave shielding film having optical transparency. The invention is not limited to the configuration but metal meshes in a predetermined pattern made of copper, nickel, aluminum, or the like may be used as the electromagnetic wave shielding film having optical transparency. By forming the electromagnetic wave shielding film as the metal meshes on the above-described optically transparent member, a scratch is suppressed and, further, damage or breakage is also suppressed. The effect appears more conspicuously than the case where the electromagnetic wave shielding film as the ITO film is used.

Although information necessary for an examination on the subject is entered by an input operation of the input unit 3 in the embodiment of the invention, the present invention is not limited to the above. It is also possible to provide an external communication interface that enables information communication with an external computer such as a workstation, and download information necessary for an examination on the subject from the external computer.

Further, in the foregoing embodiment and its modification of the invention, the electromagnetic wave shielding film on the inner wall of the casing and the electromagnetic wave shielding film having optical transparency on the inner face of the transparent film for display are electrically connected to each other via the gasket as a conductive member. However, the invention is not limited to the configuration. The electromagnetic wave shielding film on the inner wall of the casing and the electromagnetic wave shielding film having optical transparency on the inner face of the transparent film for display may be electrically connected to each other by using an adhesive in place of the gasket. For example, by using an insulating adhesive, the transparent film for display is fixed to the casing inner wall in a state where an electromagnetic wave shielding film having optical transparency on the inner face of a transparent film for display and an electromagnetic wave shielding film on the inner wall of a casing face each other and are in direct surface-contact with each other. The electromagnetic wave shielding film having optical transparency and the electromagnetic wave shielding film on the inner wall of the casing may be electrically connected to each other. Alternatively, in a state where an electromagnetic wave shielding film having optical transparency on the inner face of a transparent film for display and an electromagnetic wave shielding film on the inner wall of a casing face each other via a conductive adhesive, the transparent film for display is fixed to the casing inner wall by the conductive adhesive. The electromagnetic wave shielding film having optical transparency and the electromagnetic wave shielding film on the inner wall of the casing may be electrically connected to each other via the conductive adhesive.

In the foregoing embodiment and its modification of the invention, the electromagnetic wave shielding film having optical transparency is electrically connected to the ground via the electromagnetic wave shielding film on the inner wall of the casing and the gasket, but the invention is not limited to the configuration. The electromagnetic wave shielding film on the inner wall of the casing may be electrically connected to the ground via the electromagnetic wave shielding film having optical transparency and a conductive member such as a gasket. The electromagnetic wave shielding film having optical transparency and the electromagnetic wave shielding film on the inner wall of the casing may be electrically connected to the ground via the conductive member such as a gasket.

Further, in the embodiment and its modification of the invention, the receiving device of the capsule endoscope system having the radio communication function and the display function has been described as an example of the display device according to the invention. However, the invention is not limited to the display device. As the display device according to the invention, any display device may be used as long as it has the display function capable of displaying input information on a screen. For example, the invention can be obviously applied to various displays such as a CRT display, a liquid crystal display, and a plasma display that display input information on a screen and can be also applied to a notebook-sized or desktop computer having any of the various displays. Further, the invention can be also applied to various information devices such as PDA, cellular phone, and noncontact IC card reader/writer each having the radio communication function capable of transmitting/receiving desired information via predetermined electric waves and the display function capable of displaying input information on the screen.

As described above, in the embodiment and its modification of the invention, the electromagnetic wave shielding film is provided almost entirely on the inner wall face of the casing, and the electromagnetic wave shielding film having optical transparency is provided on the inner face of the transparent film for display provided for the display window in the casing, thereby blocking electromagnetic waves entering via the casing and the transparent film for display. Further, the electromagnetic wave shielding film and the electromagnetic wave shielding film having optical transparency are electrically connected to each other continuously along the periphery of the electromagnetic wave shielding film having optical transparency. Consequently, the series of conductive surfaces in which the conductive surface of the electromagnetic wave shielding film and the conductive surface of the electromagnetic wave shielding film having optical transparency are continuous can be formed. The inside of the casing can be covered entirely with the series of conductive surfaces. With the configuration, the display device can be realized, capable of reliably blocking the electromagnetic waves released to the casing side or the display window side, and without hindering information display to the outside via the transparent film for display, reliably preventing leakage of an electric wave from the outside into the device and leakage of an electromagnetic wave generated on the inside of the casing. In the display device, electromagnetic interference can be prevented with reliability and the adverse influence on human bodies caused by electromagnetic waves generated on the inside of the device can be also prevented.

The conductive member covering the gap between the electromagnetic wave shielding film and the electromagnetic shied film having optical transparency is provided along the periphery of the electromagnetic wave shielding film having optical transparency. The electromagnetic wave shielding film and the electromagnetic wave shielding film having optical transparency are electrically connected to each other via the conductive member. Consequently, the conductive surface of the conductive member is used as a border surface, and a series of conductive surfaces in which the conductive surface formed by the electromagnetic wave shielding film and the conductive surface formed by the electromagnetic wave shielding film having optical transparency are continuous can be formed with reliability. The inside of the casing can be covered entirely with reliability with the series of conductive surfaces. With the configuration, the display device enjoying the above-described effects and capable of more reliably preventing leakage of electromagnetic waves to the inside of the casing and to the outside can be realized.

In addition to the configuration, by also providing the radio communication function capable of receiving a radio signal from the outside, an information device can be realized, which can receive a radio signal from the outside in a good condition without being disturbed by electromagnetic waves generated on the inside of the casing, enjoy effects similar to those of the foregoing embodiment, and realize improved reception sensitivity of a radio signal from the outside. In particular, it is possible to realize the display device suitable as a receiving device for receiving image data and the like from a capsule endoscope via relatively weak electric waves in the capsule endoscope system.

### INDUSTRIAL APPLICABILITY

As described above, the display device according to the present invention is suitable as a receiving device capable of reliably preventing leakage of an electric wave from the outside into the casing and leakage of an electromagnetic wave generated on the inside of the casing to the outside without hindering information display to the outside via the transparent film for display, and particularly, having the radio communication function and the display function, in particular, a receiving device in a capsule endoscope system for receiving image data and the like from a capsule endoscope via relatively weak electric waves.

## Claims

1. A display device (1) comprising:
a shielding film (15) provided on an inner wall of a casing (12) having a display window (12a), the shielding film (15) blocking an electromagnetic wave via the casing (12); and
an optically transparent shielding film (16a; 23; 31; 41) provided on an inner surface of a transparent member (14, 16b; 22) which is provided in a frame of the display window (12a), the optically transparent shielding film (16a; 23; 31; 41) having an optical transparency, being capable of blocking an external electromagnetic wave passing through the transparent member (14, 16b; 22) and capable of preventing internal electromagnetic waves from passing through the transparent member (14, 16b, 22), and being electrically connected to the shielding film (15), and **characterized in that**,
a conductive member (17; 24; 42) covers a gap between the shielding film (15) and the optically transparent shielding film (16a; 23; 31; 41) along a periphery of the optically transparent shielding film (16a; 23; 31; 41), and electrically connects the shielding film (15) and the optically transparent shielding film (16a; 23; 31; 41), wherein
the conductive member (17; 24; 42) forms a series of conductive surfaces with the shielding film (15) and the optically transparent shielding film (16a; 23; 31; 41), and fixes the optically transparent shielding film (16a, 23; 31; 41) to an inner wall periphery around the display window (12a).

2. The display device (1) according to claim 1, wherein the transparent member (14, 16b; 22) for display comprises:
a first transparent member (14) which is fit in a frame of the display window (12a); and
a second transparent member (16b) which is fixed to the display window (12a) from an inside of the first transparent member (14) and covers the display window (12a), and
the optically transparent shielding film (16a) is provided on a back side of a side on which the second transparent member (16b) is fixed to the display window (12a).

3. The display device (1) according to claim 1, wherein the optically transparent shielding film (16a; 23; 31; 41) is an indium-tin alloy oxide film.

4. The display device (1) according to claim 1, wherein the optically transparent shielding film (16a; 23; 31; 41) is a metal mesh.

5. The display device (1) according to claim 1, further comprising a receiving unit (2a to 2h, 5, 6) that is capable of receiving image data of an inside of a subject (100) from a capsule endoscope (101) in the subject (100) via a predetermined electric wave.

## Patentansprüche

1. Anzeigegerät (1), das umfasst:
einen Abschirmfilm (15), der an einer Innenwand eines Gehäuses (12) vorgesehen ist, das ein Anzeigefenster (12a) hat, wobei der Abschirmfilm (15) eine elektromagnetische Welle durch das Gehäuse (12) blockiert; und
einen optisch transparenten Abschirmfilm (16a; 23; 31; 41), der an einer inneren Oberfläche eines transparenten Elements (14, 16b; 22) vorgesehen ist, das in einem Rahmen des Anzeigefensters (12a) vorgesehen ist, wobei der optisch transparente Abschirmfilm (16a; 23; 31; 41) eine optische Transparenz hat, dazu in der Lage ist, eine externe elektromagnetische Welle, die das transparente Element (14, 16b; 22) passiert, zu blockieren und dazu in der Lage ist, zu verhindern, dass interne elektromagnetische Wellen das transparente Element (14, 16b, 22) passieren, und elektrisch mit dem Abschirmfilm (15) verbunden ist, und
**dadurch gekennzeichnet, dass**
ein leitfähiges Element (17; 24; 42) einen Spalt zwischen dem Abschirmfilm (15) und dem optisch transparenten Abschirmfilm (16a; 23; 31; 41) entlang eines Umfangs des optisch transparenten Abschirmfilms (16a; 23; 31; 41) abdeckt und den Abschirmfilm (15) und den optisch transparenten Abschirmfilm (16a; 23; 31; 41) elektrisch verbindet, wobei
das leitfähige Element (17; 24; 42) mit dem Abschirmfilm (15) und dem optisch transparenten Abschirmfilm (16a; 23; 31; 41) eine Serie von leitfähigen Oberflächen bildet und den optisch transparenten Abschirmfilm (16a; 23; 31; 41) an einem Innenwandumfang um das Anzeigefenster (12a) fixiert.

2. Anzeigegerät (1) gemäß Anspruch 1, bei dem das transparente Element (14, 16b, 22) zum Anzeigen umfasst:
ein erstes transparentes Element (14), das in einen Rahmen des Anzeigefensters (12a) eingepasst ist; und
ein zweites transparentes Element (16b), das von einer Innenseite des ersten transparenten Elements (14) an dem Anzeigefenster (12a) fixiert ist und das Anzeigefenster (12a) abdeckt, wobei
der optisch transparente Abschirmfilm (16a) an einer Rückseite einer Seite vorgesehen ist, an welcher das zweite transparente Element (16b) an dem Anzeigefenster (12a) fixiert ist.

3. Anzeigegerät (1) gemäß Anspruch 1, wobei der optisch transparente Abschirmfilm (16a; 23; 31; 41) ein Indium-Zinn-Legierungs-Oxid-Film ist.

4. Anzeigegerät (1) gemäß Anspruch 1, wobei der optisch transparente Abschirmfilm (16a; 23; 31; 41) ein Metallgitter ist.

5. Anzeigegerät (1) gemäß Anspruch 1, das ferner eine Empfangseinheit (2a bis 2h, 5, 6) umfasst, die dazu in der Lage ist, Bilddaten eines Inneren eines Subjekts (100) von einem Kapselendoskop (101) in dem Subjekt (100) über eine vorbestimmte elektrische Welle zu empfangen.

## Revendications

1. Dispositif d'affichage (1) comprenant :
un film de protection (15) prévu sur une paroi intérieure d'un boîtier (12) ayant une fenêtre d'affichage (12a), le film de protection (15) bloquant une onde électromagnétique par l'intermédiaire du boîtier (12) ; et
un film de protection optiquement transparent (16a ; 23 ; 31 ; 41) prévu sur une surface intérieure d'un élément transparent (14, 16b ; 22) qui est prévu dans un cadre de la fenêtre d'affichage (12a), le film de protection optiquement transparent (16a ; 23 ; 31 ; 41) ayant une transparence optique, étant apte à bloquer une onde électromagnétique externe passant à travers l'élément transparent (14, 16b ; 22) et apte à empêcher que des ondes électromagnétiques internes ne passent à travers l'élément transparent (14, 16b, 22), et étant électriquement connecté au film de protection (15), et **caractérisé en ce que**
un élément conducteur (17 ; 24 ; 42) recouvre un espace entre le film de protection (15) et le film de protection optiquement transparent (16a ; 23 ; 31 ; 41) le long d'une périphérie du film de protection optiquement transparent (16a ; 23 ; 31 ; 41), et connecte électriquement le film de protection (15) et le film de protection optiquement transparent (16a ; 23 ; 31 ; 41), dans lequel
l'élément conducteur (17 ; 24 ; 42) forme une série de surfaces conductrices avec le film de protection (15) et le film de protection optiquement transparent (16a ; 23 ; 31 ; 41), et fixe le film de protection optiquement transparent (16a ; 23 ; 31 ; 41) sur une périphérie de paroi intérieure autour de la fenêtre d'affichage (12a).

2. Dispositif d'affichage (1) selon la revendication 1, dans lequel l'élément transparent (14, 16b ; 22) pour affichage comprend :
un premier élément transparent (14) qui est ajusté dans un cadre de la fenêtre d'affichage (12a) ; et
un deuxième élément transparent (16b) qui est fixé à la fenêtre d'affichage (12a) à partir d'un intérieur du premier élément transparent (14) et recouvre la fenêtre d'affichage (12a), et
le film de protection optiquement transparent (16a) est prévu sur un côté arrière d'un côté sur lequel le deuxième élément transparent (16b) est fixé à la fenêtre d'affichage (12a).

3. Dispositif d'affichage (1) selon la revendication 1, dans lequel le film de protection optiquement transparent (16a ; 23 ; 31 ; 41) est un film d'oxyde d'alliage indium-étain.

4. Dispositif d'affichage (1) selon la revendication 1, dans lequel le film de protection optiquement transparent (16a ; 23 ; 31 ; 41) est un maillage métallique.

5. Dispositif d'affichage (1) selon la revendication 1, comprenant en outre une unité de réception (2a à 2h, 5, 6) qui est apte à recevoir des données d'image d'un intérieur d'un sujet (100) à partir d'un endoscope de type capsule (101) dans le sujet (100) par l'intermédiaire d'une onde électrique prédéterminée.
